(19)

**Europäisches Patentamt**
**European Patent Office**
**Office européen des brevets**

(11) **EP 3 868 365 A1**

(12) **EUROPEAN PATENT APPLICATION**

(43) Date of publication:
25.08.2021 Bulletin 2021/34

(51) Int Cl.:
*A61K 9/20* (2006.01)      *A61K 47/02* (2006.01)
*A61K 47/20* (2006.01)     *A61K 47/38* (2006.01)
*A61K 47/36* (2006.01)

(21) Application number: 20217247.4

(22) Date of filing: 24.12.2020

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.02.2020 TW 109105282**

(71) Applicant: **Taiwan Sunpan Biotechnology Development Co., Ltd.**
**Taoyuan City 33045 (TW)**

(72) Inventors:
• **LEE, Sen-Bin**
**33045 Taoyuan City (TW)**
• **Li, Yi-Ling**
**33045 Taoyuan City (TW)**
• **Li, Chiao-Ling**
**33045 Taoyuan City (TW)**

(74) Representative: **Gilani, Anwar**
**Venner Shipley LLP**
**Byron House**
**Cambridge Business Park**
**Cowley Road**
**Cambridge CB4 0WZ (GB)**

(54) **PHARMACEUTICAL COMPOSITION CONTAINING ACETONE-EXTRACTED PRODUCT FROM GAMBOGE RESIN, AND FORMULATION MANUFACTURED FROM SUCH COMPOSITION**

(57)     A pharmaceutical composition includes, based on the total weight of the composition, 5% to 30% of an active pharmaceutical ingredient including an acetone-extracted product and a saccharide compound in a weight ratio of 9: 1, 20% to 75% of a solubilizing agent, 5% to 30% of a disintegrating agent, 5% to 30% of a filling agent, 0.1% to 5% of a flow agent, and 0.1% to 5% of a lubricant. The active pharmaceutical ingredient has an average particle size of about 1 $\mu$m to 30 $\mu$m. A formulation manufactured from the pharmaceutical composition is also disclosed.

EP 3 868 365 A1

**Description**

The present disclosure relates to a pharmaceutical composition containing an acetone-extracted product from gamboge resin. In particular, the pharmaceutical composition can be manufactured into a solid dosage form by dry granulation.

Gamboge resin is the gum-resin secreted by the plant of *Garcinia sp.* of the family Guttiferae. It has been used as a source of vegetative dyes and pigments since the old days. It is also used in folk medicine in some areas such as India and Thailand.

Garcinia (TENGHUANG in pinyin), which is commonly known as gamboge, is a kind of evergreen trees that grow in tropical regions. The main species grown in India is "*Garcinia morella* Desv," whereas the main species grown in Thailand is "G. *harburyi* Hook f." Before the flowering period, the bark of the tree is cut open in a spiral shape about 2 meters from the ground to collect the exuding resin. The resin is then subjected to heat-drying to result in a solidified gamboge resin.

According to traditional Chinese medicine (TCM), gamboge is effective in combating inflammations, clearing away toxins, stopping blood bleeding, and killing worms . Ever since 1934, there have been a number of reports on the components of the gamboge resin. At present, it is known that many compounds can be isolated from extracts of gamboge resin, including: morellin, morellic acid, gambogic acid, morellinol, isomorellin, isomorellic acid, isogambogic acid, isomorellinol, neogambogic acid, desoxymorellin, dihydroisomorellin, $\alpha$-guttiferin, $\beta$-guttiferin, gambogenic acid, desoxyg-ambogenin, gambogellic acid, epigambogic acid, epiisogambogic acid, isogambogenic acid, 30-hydroxygambogic acid, etc.

It has been reported that an acetone-extracted product of gamboge resin and the compounds obtained from such product have activities in inhibiting the growth of tumor/cancer cells and have analgesic and anti-inflammatory effects. For instance, US 7,138,428 B2 discloses an acetone-extracted product from gamboge resin, i.e., TSB-14. In addition, as disclosed in US 7, 138, 428 B2, nine compounds were further purified from said acetone-extracted product TSB-14, including, at the time, a new compound formoxanthone A, and eight known compounds betulin, betulinic acid, morellic acid, isomorellic acid, gambogic acid, isogambogic acid, isomorellinol and desoxymorellin. The acetone-extracted product TSB-14 and the nine purified compounds have been demonstrated to have effects in inhibiting the growth of tumor/cancer cells such as liver cancer cells (HepG2), lung cancer cells (A549), breast cancer cells (MCF-7), colon cancer cells (HT-29), leukemia cells (HL-60), and lymphoma cancer cells (U937).

US 2011/0305784 A1 discloses, at the time, seventeen new compounds and five fractionated products obtained from an acetone-extracted product of gamboge resin (i.e., TSB-14). The seventeen new compounds and the five fractionated products have been demonstrated to have activities in inhibiting the growth of tumor/cancer cells. In addition, the acetone-extracted product TSB-14 and the five fractionated products obtained therefrom have been demonstrated to have analgesic and anti-inflammatory effects.

Granulation is a process in which small particles agglomerate into larger, multi-particle masses called granules. The granulation process is routinely utilized in the pharmaceutical industry for formulating solid oral dosage formulations, as well as in various other industries. Granulation has several advantages such as reducing dust of fine particles that might cause potential health and environmental hazards, avoiding segregation of different components in a formulation, producing granules that are easy to handle and transport, and improving flowability and compressibility of the ingredients.

Granulation processes can be divided into two types : wet granulation and dry granulation. Wet granulation processes utilize some form of solvent or liquid binder to bind small particles together to form agglomerates. Wet granulation may use any of low-shear mixing, high-shear mixing, extrusion-spheronization, or fluid-bed processing for producing wet granules, which are then dried, sieved, and optionally ground prior to being compressed into tablets (when tableting is desired). Wet granulation is frequently used in the pharmaceutical industry, but it has proven to have some disadvantages. For example, the solvent or liquid binder may have an adverse effect on other ingredients in the formulation and/or on one or more properties of the end product, such as a tablet. Further, the wet granulation process usually requires a drying step to remove the solvent or liquid binder after granulation.

Dry granulation processes are carried out without a solvent or liquid binder. Dry granulation can be employed to overcome some of the disadvantages of wet granulation that result from the use of a solvent or liquid binder. In a dry granulation process, powdered components, typically in the form of fine particles, are mixed prior to granulation and then compressed to yield hard granules which may then be ground and sieved, as necessary, to produce particles of a desired size distribution. Dry granulation may use either slugging or roller compression to produce compacts, also known as briquettes, flakes or ribbons, which may then be milled to obtain the desired granules.

The applicant has attempted to develop a new pharmaceutical composition containing the acetone-extracted product TSB-14 as disclosed in US 7,138,428 B2, which is suitable for a dry granulation process.

Accordingly, the present disclosure provides a pharmaceutical composition comprising, based on the total weight of the composition:

5% to 30% of an active pharmaceutical ingredient including an acetone-extracted product and a saccharide compound in a weight ratio of 9:1, the active pharmaceutical ingredient having an average particle size of about 1 $\mu$m to 30 $\mu$m;

20% to 75% of a solubilizing agent;

5% to 30% of a disintegrating agent;

5% to 30% of a filling agent;

0.1% to 5% of a flow agent; and

0.1% to 5% of a lubricant;

wherein the acetone-extracted product is obtained by pulverizing gamboge resin into powder, followed by subjecting the pulverized powder to extraction with acetone.

The present disclosure also provides a solid dosage formulation which is manufactured from a pharmaceutical composition as described above.

It is to be understood that, if any prior art publication is referred to herein, such reference does not constitute an admission that the publication forms a part of the common general knowledge in the art, in Taiwan or any other country.

For the purpose of this specification, it will be clearly understood that the word "comprising" means "including but not limited to", and that the word "comprises" has a corresponding meaning.

Unless defined otherwise, all technical and scientific terms used herein have the meaning commonly understood by a person skilled in the art to which the present disclosure belongs. One skilled in the art will recognize many methods and materials similar or equivalent to those described herein, which could be used in the practice of the present disclosure. Indeed, the present disclosure is in no way limited to the methods and materials described.

For the purposes of this specification and appended claims, unless otherwise indicated, all numbers expressing amounts, sizes, dimensions, proportions, shapes, formulations, parameters, percentages, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error and the like, and other factors known to those of skill in the art depending on the desired properties sought to be obtained by the presently disclosed subject matter. For example, the term "about," when referring to a value can be meant to encompass variations of, in some aspects, $\pm$ 100% in some aspects $\pm$ 50%, in some aspects $\pm$ 20%, in some aspects $\pm$ 10%, in some aspects $\pm$ 5%, in some aspects $\pm$1%, in some aspects $\pm$ 0.5%, and in some aspects $\pm$ 0.1% from the specified amount, as such variations are appropriate to perform the disclosed methods or employ the disclosed compositions.

As used herein, the terms "active pharmaceutical ingredient", "active component", or "biologically active compound" are used interchangeably, and are understood to include any substance or material, or combination of substances and materials, which is pharmacologically active and, hence, has therapeutic value.

In Example 1 of US 7,138,428 B2, the applicant disclosed an acetone-extracted product from gamboge resin, i.e., TSB-14, which was obtained by pulverizing gamboge resin into powder, followed by subjecting the pulverized powder to extraction with acetone. Through further research, the applicant surprisingly found that a pharmaceutical composition containing the acetone-extracted product TSB-14 can be compacted by dry granulation, and the slugs thus obtained can be milled to produce particles of desired sizes. The resultant particles can be formulated into a tablet, and the tablet exhibits excellent drug release characteristics.

Therefore, the present disclosure provides a pharmaceutical composition comprising, based on the total weight of the composition:

5% to 30% of an active pharmaceutical ingredient including an acetone-extracted product and a saccharide compound in a weight ratio of 9:1, the active pharmaceutical ingredient having an average particle size of about 1 $\mu$m to 30 $\mu$m;

20% to 75% of a solubilizing agent;

5% to 30% of a disintegrating agent;

5% to 30% of a filling agent;

0.1% to 5% of a flow agent; and

0.1% to 5% of a lubricant;

wherein the acetone-extracted product is obtained by pulverizing gamboge resin into powder, followed by subjecting the pulverized powder to extraction with acetone.

In an exemplary embodiment of the present disclosure, the active pharmaceutical ingredient has an average particle size of about 10 $\mu$m.

According to the present disclosure, the saccharide compound may be selected from the group consisting of glucose, lactose, sucrose, brown sugar, sorbitol, mannitol, starch, and combinations thereof. In an exemplary embodiment, the saccharide compound is brown sugar.

According to the present disclosure, the solubilizing agent may be selected from the group consisting of sodium lauryl sulfate, sodium cetearyl sulfate, dioctyl sodium sulfosuccinate, carnauba wax, benzalkonium chloride, cetylpyridinium chloride, polyoxyethylene alkyl ether, polyethylene glycol stearate, polyethylene glycol-6 (PEG-6) caprylic/capric glycerides, lauroyl polyoxylglycerides, caprylocaproyl polyoxylglycerides, linoleoyl polyoxylglycerides, oleoyl polyoxylglycerides, stearoyl polyoxylglycerides, polyoxyethylene sorbitan monolaurate (Tween 20), polyoxyethylene sorbitan monopalmitate (Tween 40), polyoxyethylene sorbitan monostearate (Tween 60), polyoxyethylene sorbitan monooleate (Tween 80), sorbitan monoisostearate, sorbitan monolaurate, sorbitan monooleate, sorbitan monopalmitate, sorbitan monostearate, sorbitan sesquistearate, sorbitan sesquioleate, sorbitan sesquiisostearate, sorbitan trilaurate, sorbitan trioleate, sorbitan tristearate, and combinations thereof. In an exemplary embodiment, the solubilizing agent is sodium lauryl sulfate.

According to the present disclosure, the disintegrating agent may be selected from the group consisting of alginic acid, calcium alginate, calcium carboxymethyl cellulose, sodium carboxymethyl cellulose, cellulose, chitosan, pregelatinized starch, cross-linked sodium carboxymethyl cellulose, cross-linked polyvinylpyrrolidone, glycine, guar gum, hydroxypropyl cellulose, low-substituted hydroxypropyl cellulose, magnesium aluminum silicate, methyl cellulose, polacrilin potassium, polyvinylpyrrolidone, sodium alginate, sodium carboxymethyl starch, partially pregelatinized starch, and combinations thereof. In an exemplary embodiment, the disintegrating agent is cross-linked sodium carboxymethyl cellulose.

According to the present disclosure, the filling agent may be selected from the group consisting of aluminum alginate, calcium carbonate, calcium lactate, tricalcium phosphate, calcium hydrogen phosphate, calcium hydrogen phosphate dihydrate, calcium silicate, calcium sulfate, silicified microcrystalline cellulose, cellulose acetate, dextrin, erythritol, ethyl cellulose, fructose, fumaric acid, isomalt, kaolin, lactitol, magnesium carbonate, maltodextrin, medium chain triglyceride, microcrystalline cellulose, polydextrose, polymethyl acrylate, simethicone, sodium chloride, corn starch, sugar spheres, 2-hydroxypropyl-$\beta$-cyclodextrin, arabic gum, fucose, xylitol, and combinations thereof. In an exemplary embodiment, the filling agent is microcrystalline cellulose.

According to the present disclosure, the flow agent may be selected from the group consisting of magnesium oxide, magnesium silicate, magnesium trisilicate, silicon dioxide (e.g. colloidal silicon dioxide such as hydrophobic colloidal silicon dioxide), and combinations thereof. In an exemplary embodiment, the flow agent is silicon dioxide.

According to the present disclosure, the lubricant may be selected from the group consisting of magnesium stearate, calcium stearate, monostearin, glyceryl behenate, glyceryl palmitate, glyceryl stearate, magnesium dodecyl sulfate, myristic acid, palmitic acid, Poloxamer 188, Poloxamer 237, Poloxamer 338, Poloxamer 407, polyethylene glycol 1000 (PEG 1000), polyethylene glycol 1450 (PEG 1450), polyethylene glycol 1540 (PEG 1540), polyethylene glycol 2000 (PEG 2000), polyethylene glycol 3000 (PEG 3000), polyethylene glycol 3350 (PEG 3350), polyethylene glycol 4000 (PEG 4000), polyethylene glycol 4600 (PEG 4600), polyethylene glycol 8000 (PEG 8000), sodium benzoate, sodium stearyl fumarate, stearic acid, talc, zinc stearate, potassium stearate, and combinations thereof. In an exemplary embodiment, the lubricant is magnesium stearate.

In an exemplary embodiment of the present disclosure, the pharmaceutical composition may comprise, based on the total weight of the composition, 5% of the active pharmaceutical ingredient, 75% of sodium lauryl sulfate, 9.9% of cross-linked sodium carboxymethyl cellulose, 9.9% of microcrystalline cellulose, 0.1% of silicon dioxide, and 0.1% of magnesium stearate.

In another exemplary embodiment of the present disclosure, the pharmaceutical composition may comprise, based on the total weight of the composition, 5% of the active pharmaceutical ingredient, 25% of sodium lauryl sulfate, 30% of cross-linked sodium carboxymethyl cellulose, 30% of microcrystalline cellulose, 5% of silicon dioxide, and 5% of magnesium stearate.

In yet another exemplary embodiment of the present disclosure, the pharmaceutical composition may comprise, based on the total weight of the composition, 23.8% of the active pharmaceutical ingredient, 47.6% of sodium lauryl sulfate, 15.7% of cross-linked sodium carboxymethyl cellulose, 11.9% of microcrystalline cellulose, 0.5% of silicon dioxide, and 0.5% of magnesium stearate.

According to the present disclosure, the pharmaceutical composition may be made into a dosage form suitable for oral administration using technology well-known to those skilled in the art. Examples of the dosage form include, but are not limited to, a tablet (e.g. a coated tablet), a granule, a powder, a capsule (e.g. a coated capsule and a pellet-filled capsule), a pellet, and a pill. In an exemplary embodiment of the present disclosure, the pharmaceutical composition is manufactured into a tablet.

According to the present disclosure, the pharmaceutical composition may be manufactured into a tablet using direct compression or dry granulation well-known to those skilled in the art.

In certain embodiments, the pharmaceutical composition may be manufactured into a tablet using dry granulation. There are two dry granulation techniques commonly used in the pharmaceutical industry, including slugging and roll compaction. The pharmaceutical composition may be converted into slugs by means of flat-faced punches or roller compactors. The slugs are then screened or milled into granules for tableting.

In an exemplary embodiment of the present disclosure, the pharmaceutical composition is manufactured into a tablet by slugging.

In view of the foregoing, the present disclosure also provides a solid dosage formulation which is manufactured from the aforesaid pharmaceutical composition. The solid dosage formulation may be in any of the dosage forms mentioned above, and may be manufactured using the techniques addressed above.

The disclosure will be further described by way of the following examples. However, it should be understood that the following examples are solely intended for the purpose of illustration and should not be construed as limiting the disclosure in practice.

## EXAMPLES

### General Experimental Materials:

1. The ingredients used for preparation of the tablet containing the acetone-extracted product TSB-14 according to the present disclosure are listed in Table 1.

Table 1

| Ingredients | Sources |
|---|---|
| Sodium lauryl sulfate (SLS) | BASF Co., Ltd. |
| Cross-linked sodium carboxymethyl cellulose | FMC BioPolymer Co., Ltd. |
| Microcrystalline cellulose 102 | Wei Ming Pharmaceutical Mfg. Co., Ltd., Taiwan |
| Silicon dioxide | PQ Corporation |
| Magnesium stearate | Peter Greven GmbH & Co. |

2. The acetone-extracted product TSB-14 used in the examples was prepared according to Example 1 of US 7,138,428 B2.

### Example 1. Preparation of tablet containing acetone-extracted product TSB-14

Seven tablets containing the acetone-extracted product TSB-14 were prepared using the recipe shown in Table 2, and the preparation processes of these seven tablets are described below. Even though the preparation processes of all the tablets are the same, as shown in Table 2, only tablets 1 and 3 to 7 were manufactured from the pharmaceutical composition of the present disclosure having the required compositional ratios.

Table 2

| Ingredients | Tablet 1 | Tablet 2 | Tablet 3 | Tablet 4 | Tablet 5 | Tablet 6 | Tablet 7 |
|---|---|---|---|---|---|---|---|
| | mg/tablet | | | | | | |
| TSB-9-W1 | 100 (23.8%) | 100 (23.8%) | 21 (5%) | 21 (5%) | 84 (20%) | 42 (10%) | 126 (30%) |
| Sodium lauryl sulfate (SLS) | 200 (47.6%) | 100 (23.8%) | 105 (25%) | 315 (75%) | 252 (60%) | 168 (40%) | 251 (59.8%) |
| Cross-linked sodium carboxymethyl cellulose | 66 (15.7%) | 66 (15.7%) | 126 (30%) | 42 (9.9%) | 21 (5%) | 84 (20%) | 21 (5%) |
| Microcrystalline cellulose 102 | 50 (11.9%) | 150 (35.7%) | 126 (30%) | 42 (9.9%) | 21 (5%) | 84 (20%) | 21 (5%) |
| Silicon dioxide | 2 (0.5%) | 2 (0.5%) | 21 (5%) | 0.42 (0.1%) | 21 (5%) | 21 (5%) | 0.42 (0.1%) |

(continued)

| Ingredients | Tablet 1 | Tablet 2 | Tablet 3 | Tablet 4 | Tablet 5 | Tablet 6 | Tablet 7 |
|---|---|---|---|---|---|---|---|
| | mg/tablet | | | | | | |
| Magnesium stearate | 2 (0.5%) | 2 (0.5%) | 21 (5%) | 0.42 (0.1%) | 21 (5%) | 21 (5%) | 0.42 (0.1%) |
| Total Weight | 420 | | | | | | |

The percentage of the respective ingredient indicated in the parentheses is the weight percentage relative to the total weight of the tablet.

The acetone-extracted product TSB-14 was mixed with brown sugar in a ratio of 9:1 (wt/wt), followed by milling the resultant mixture, so as to obtain a formulation TSB-9-W1 (i.e., an active pharmaceutical ingredient (API)) having an average particle size of about 10 $\mu$m. A respective one of the API, SLS, cross-linked sodium carboxymethyl cellulose, microcrystalline cellulose 102, and silicon dioxide was sieved using a 20 mesh sieve (mesh NO. 20, Kuang Yang), and magnesium stearate was sieved using a 40 mesh sieve (mesh NO. 40, Kuang Yang). Thereafter, the API, SLS, cross-linked sodium carboxymethyl cellulose, microcrystalline cellulose 102, and silicon dioxide were added into a grinding mill, followed by blending therein for 5 minutes. Magnesium stearate was subsequently added into the grinding mill, followed by blending therein for 5 minutes. Dry granulation of the resultant mixture was performed using a dry granulator.

The slugs thus obtained had a density between 0.4 and 0.55 g/cm$^3$. The slugs were then milled into particles . The resultant particles were screened first through a 20 mesh sieve and subsequently through a 40 mesh sieve. The particles which could pass through the 20 mesh sieve but be retained on the 40 mesh sieve were collected. The collected particles were compressed into tablets using a tablet machine (CB-75A, Chuang Pao Special Precision Industry CO., LTD.). The resultant tablets had a size of 0.6 cm (width) x 1.4 cm (length) x 0.57 cm (thickness) and a weight of 420 mg.

**Example 2. Dissolution test of tablet containing the acetone-extracted product TSB-14**

In order to evaluate the release rates of the API from tablets 1-7 obtained in Example 1, the following analyses were conducted.

***Methods:***

The *in vitro* release rates of the API from tablets 1-7 (n=6 for each tablet) were evaluated using a USP dissolution apparatus II machine (RC806D, Tianda Tianfa Technology Co., Ltd, China) . A respective tablet was placed in 900 mL of pure water at 37 $\pm$ 0.5 °C and agitated at a paddle speed of 75 rpm for 80 minutes. The respective dissolution solution was collected, and was subsequently filtered using a filter with a porosity of 0.45 $\mu$m. The test solution thus obtained was then subjected to determination of absorbance at a wavelength of 360 nm ($A_{360}$) using a UV-VIS spectrophotometer (SP-8001, Shishin Technology Co., Ltd, Taiwan) . 900 mL of pure water was used as a blank control. 100 mg of the formulation TSB-9-W1 was dissolved in 900 mL of pure water, and then was used as a positive control. The blank and positive controls were also subjected to determination of $A_{360}$.

The release rate (%) of the API from each tablet was calculated using the following Equation (I):

$$\text{Release rate (\%)} = [(A-C)/(B-C)] \times 100 \quad (I)$$

wherein A=$A_{360}$ value of the test solution;
B=$A_{360}$ value of the positive control; and
C=$A_{360}$ value of the blank control.

***Results:***

As shown in Table 3 below, the release rate of the API of each of tablets 1 and 3-7 (manufactured from the pharmaceutical composition of the present disclosure) was significantly higher than that of tablet 2 (manufactured from a pharmaceutical composition different from that of the present disclosure), indicating that the pharmaceutical composition of the present disclosure can effectively enhance the release of the acetone-extracted product TSB-14.

Table 3

| Sample | Release rate (%) |
|---|---|
| Tablet 1 | 96.32 |
| Tablet 2 | 22.1 |
| Tablet 3 | 98.2 |
| Tablet 4 | 100.12 |
| Tablet 5 | 95.63 |
| Tablet 6 | 96.24 |
| Tablet 7 | 75.2 |

The experimental results reveal that the pharmaceutical composition of the present disclosure can be compacted by dry granulation, and the slugs thus obtained can be milled to produce particles of desired sizes. The resultant particles can be formulated into a solid dosage form (e.g. a tablet, a granule, a powder, a capsule, a pellet, or a pill), and such solid dosage form can exhibit excellent drug release characteristics.

In the description above, for the purposes of explanation, numerous specific details have been set forth in order to provide a thorough understanding of the embodiments. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects, and that one or more features or specific details from one embodiment may be practiced together with one or more features or specific details from another embodiment, where appropriate, in the practice of the disclosure.

## Claims

1. A pharmaceutical composition **characterized by** comprising, based on the total weight of the composition:

   5% to 30% of an active pharmaceutical ingredient including an acetone-extracted product and a saccharide compound in a weight ratio of 9:1, the active pharmaceutical ingredient having an average particle size of about 1 $\mu$m to 30 $\mu$m;
   20% to 75% of a solubilizing agent;
   5% to 30% of a disintegrating agent;
   5% to 30% of a filling agent;
   0.1% to 5% of a flow agent; and
   0.1% to 5% of a lubricant;

   the acetone-extracted product being obtained by pulverizing gamboge resin into powder, followed by subjecting the pulverized powder to extraction with acetone.

2. The pharmaceutical composition as claimed in Claim 1, **characterized in that** the solubilizing agent is selected from the group consisting of sodium lauryl sulfate, sodium cetearyl sulfate, dioctyl sodium sulfosuccinate, carnauba wax, benzalkonium chloride, cetylpyridinium chloride, polyoxyethylene alkyl ether, polyethylene glycol stearate, polyethylene glycol-6 caprylic/capric glycerides, lauroyl polyoxylglycerides, caprylocaproyl polyoxylglycerides, linoleoyl polyoxylglycerides, oleoyl polyoxylglycerides, stearoyl polyoxylglycerides, polyoxyethylene sorbitan monolaurate, polyoxyethylene sorbitan monopalmitate, polyoxyethylene sorbitan monostearate, polyoxyethylene sorbitan monooleate, sorbitan monoisostearate, sorbitan monolaurate, sorbitan monooleate, sorbitan monopalmitate, sorbitan monostearate, sorbitan sesquistearate, sorbitan sesquioleate, sorbitan sesquiisostearate, sorbitan trilaurate, sorbitan trioleate, sorbitan tristearate, and combinations thereof.

3. The pharmaceutical composition as claimed in Claim 1 or 2, **characterized in that** the disintegrating agent is selected from the group consisting of alginic acid, calcium alginate, calcium carboxymethyl cellulose, sodium carboxymethyl cellulose, cellulose, chitosan, pregelatinized starch, cross-linked sodium carboxymethyl cellulose, cross-linked polyvinylpyrrolidone, glycine, guar gum, hydroxypropyl cellulose, low-substituted hydroxypropyl cellulose, magnesium aluminum silicate, methyl cellulose, polacrilin potassium, polyvinylpyrrolidone, sodium alginate, sodium carboxymethyl starch, partially pregelatinized starch, and combinations thereof.

4. The pharmaceutical composition as claimed in any one of Claims 1 to 3, **characterized in that** the filling agent is selected from the group consisting of aluminum alginate, calcium carbonate, calcium lactate, tricalcium phosphate, calcium hydrogen phosphate, calcium hydrogen phosphate dihydrate, calcium silicate, calcium sulfate, silicified microcrystalline cellulose, cellulose acetate, dextrin, erythritol, ethyl cellulose, fructose, fumaric acid, isomalt, kaolin, lactitol, magnesium carbonate, maltodextrin, medium chain triglyceride, microcrystalline cellulose, polydextrose, polymethyl acrylate, simethicone, sodium chloride, corn starch, sugar spheres, 2-hydroxypropyl-$\beta$-cyclodextrin, arabic gum, fucose, xylitol, and combinations thereof.

5. The pharmaceutical composition as claimed in any one of Claims 1 to 4, **characterized in that** the flow agent is selected from the group consisting of magnesium oxide, magnesium silicate, magnesium trisilicate, silicon dioxide, and combinations thereof.

6. The pharmaceutical composition as claimed in any one of Claims 1 to 5, **characterized in that** the lubricant is selected from the group consisting of magnesium stearate, calcium stearate, monostearin, glyceryl behenate, glyceryl palmitate, glyceryl stearate, magnesium dodecyl sulfate, myristic acid, palmitic acid, Poloxamer 188, Poloxamer 237, Poloxamer 338, Poloxamer 407, polyethylene glycol 1000, polyethylene glycol 1450, polyethylene glycol 1540, polyethylene glycol 2000, polyethylene glycol 3000, polyethylene glycol 3350, polyethylene glycol 4000, polyethylene glycol 4600, polyethylene glycol 8000, sodium benzoate, sodium stearyl fumarate, stearic acid, talc, zinc stearate, potassium stearate, and combinations thereof.

7. The pharmaceutical composition as claimed in any one of Claims 1 to 6, **characterized in that** the saccharide compound is selected from the group consisting of glucose, lactose, sucrose, brown sugar, sorbitol, mannitol, starch, and combinations thereof.

8. The pharmaceutical composition as claimed in any one of Claims 1 to 7, **characterized in that** the saccharide compound is brown sugar.

9. The pharmaceutical composition as claimed in any one of Claims 1 to 8, which is manufactured into a solid dosage form selected from the group consisting of a tablet, a granule, a powder, a capsule, a pellet, or a pill.

10. A solid dosage formulation which is manufactured from a pharmaceutical composition as claimed in any one of Claims 1 to 9.

11. The solid dosage formulation as claimed in Claim 10, which is in a form selected from the group consisting of a tablet, a granule, a powder, a capsule, a pellet, or a pill.

Europäisches
Patentamt

European
Patent Office

Office européen
des brevets

# EUROPEAN SEARCH REPORT

**Application Number**

EP 20 21 7247

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2005/261363 A1 (LEE SEN-BIN [TW] ET AL) 24 November 2005 (2005-11-24) * paragraph [0066] - paragraph [0069]; claims 20-23; table 4 * ----- | 1-11 | INV. A61K9/20 A61K47/02 A61K47/20 A61K47/38 A61K47/36 |
| Y | EP 2 395 006 A1 (TAIWAN SUNPAN BIOTECHNOLOGY DEV CO LTD [TW]) 14 December 2011 (2011-12-14) * paragraphs [0029], [0068] - [0071]; claim 2 * ----- | 1-11 | |

TECHNICAL FIELDS
SEARCHED       (IPC)

A61K

The present search report has been drawn up for all claims

| Place of search | Date of completion of the search | Examiner |
|---|---|---|
| Munich | 7 June 2021 | Wörth, Christian |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...........................................................................................
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 20 21 7247

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2021

| Patent document cited in search report | | | Publication date | Patent family member(s) | | Publication date |
|---|---|---|---|---|---|---|
| US 2005261363 | A1 | | 24-11-2005 | JP | 4425109 B2 | 03-03-2010 |
| | | | | JP | 2005330261 A | 02-12-2005 |
| | | | | JP | 2009280610 A | 03-12-2009 |
| | | | | TW | I282280 B | 11-06-2007 |
| | | | | US | 2005261363 A1 | 24-11-2005 |
| EP 2395006 | A1 | | 14-12-2011 | EP | 2395006 A1 | 14-12-2011 |
| | | | | EP | 2395007 A1 | 14-12-2011 |
| | | | | EP | 2684882 A1 | 15-01-2014 |
| | | | | EP | 2684883 A1 | 15-01-2014 |
| | | | | EP | 2684884 A1 | 15-01-2014 |
| | | | | EP | 2684885 A1 | 15-01-2014 |
| | | | | HK | 1163086 A1 | 07-09-2012 |
| | | | | HK | 1163087 A1 | 07-09-2012 |
| | | | | JP | 5441947 B2 | 12-03-2014 |
| | | | | JP | 5558408 B2 | 23-07-2014 |
| | | | | JP | 2011256161 A | 22-12-2011 |
| | | | | JP | 2011256162 A | 22-12-2011 |
| | | | | TW | 201143764 A | 16-12-2011 |
| | | | | US | 2011305784 A1 | 15-12-2011 |
| | | | | US | 2011306658 A1 | 15-12-2011 |
| | | | | US | 2016089357 A1 | 31-03-2016 |
| | | | | US | 2016338991 A1 | 24-11-2016 |

EPO FORM P0459

For more details about this annex : see Official Journal of the European Patent Office, No. 12/82

**EP 3 868 365 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7138428 B2
- US 20110305784 A1